# EUROPEAN PATENT APPLICATION

(11) **EP 4 851 630 A1**
(43) Date of publication of application: **09.12.2026**
(21) Application number: 25382566.5
(22) Date of filing: 03.06.2025
(51) Int. Cl.: G01N 33/564

(54) **METHODS FOR PREDICTION OF PROGRESSION IN MULTIPLE SCLEROSIS**

(71) Applicant: Institut de Recerca Biomèdica de Lleida Fundació Dr Pifarré, 25198 Lleida (ES); Fundación Instituto de Estudios de Ciencias de la Salud de Castilla y León, 42002 Soria (ES); Universitat de Lleida, 25003 Lérida (ES)
(72) Inventor: GONZÁLEZ MINGOT, Cristina, 25198 Lleida (ES); BRIEVA RUIZ, Luis, 25198 Lleida (ES); TORRES CABESTANY, Pascual, 25001 Lleida (ES); GONZALO BENITO, Hugo, 42002 Soria (ES); GIL SÁNCHEZ, Anna Rita, 25198 Lleida (ES)
(74) Representative: Ungria López, Javier

(57) **Abstract**

The present disclosure provides a method for predicting the long-term progression of multiple sclerosis (MS), a complex and multifaceted disease characterized by immune-mediated mechanisms, chronic inflammation, demyelination, and neurodegeneration affecting the central nervous system (CNS). MS exhibits diverse clinical manifestations, and the disease course in affected individuals may range from mild to aggressive. A key aspect of MS progression is long-term disability, which is predominantly unrelated to the occurrence of clinical relapses. This phenomenon, termed progression independent of relapse activity (PIRA), has been associated with neurodegenerative processes in relapsing-remitting MS (RRMS), underscoring the need for advanced methodologies to predict and manage disease progression effectively. The disclosure also encompasses a method for classifying individuals diagnosed with MS based on their disease characteristics and progression patterns.

## Description

### TECHNICAL FIELD

The present disclosure relates to the field of immunology and the study of chronic neurological disorders. Specifically the dislosure relates to the field of multiple sclerosis (MS). More specifically, it addresses innovative methods for predicting the prognosis of MS in affected individuals. The disclosure focuses on utilizing molecular biomarkers and clinical variables to provide an accurate and personalized predictions regarding the long-term progression of MS. Furthermore, the present disclosure introduces methods for classifying individuals with MS into distinct categories based on the severity and progression of their disease.

### BACKGROUND OF THE INVENTION

Multiple sclerosis (MS) is a complex and multifaceted neurological disorder characterized by its immune-mediated, chronic inflammatory, demyelinating, and neurodegenerative nature. This disorder primarily affects the central nervous system (CNS), including the brain, spinal cord, and optic nerves, leading to a broad spectrum of clinical manifestations. The clinical course of MS is highly variable among individuals, encompassing motor, sensory, cognitive, and autonomic dysfunctions. MS predominantly affects individuals between the ages of 20 and 40; however, its epidemiological profile continues to evolve globally, owing to advancements in the understanding of its immunopathogenesis, natural disease history, and diagnostic methodologies (Karussis D. The diagnosis of multiple sclerosis and the various related demyelinating syndromes: a critical review. J Autoimmun. 2014; 48-49:134-142; Zéphir H. Progress in understanding the pathophysiology of multiple sclerosis. Rev Neurol (Paris). 2018; 174(6):358-363; Yamout B. Update in multiple sclerosis. eNeurologicalSci. 2025 Jan 11; 38:100553).

Current research highlights the multifactorial etiology of MS, involving a complex interplay of environmental and genetic factors. Among the environmental contributors, a ground breaking study has identified Epstein-Barr virus (EBV) infection as a key determinant in MS pathogenesis. The study revealed that EBV infection elevates the risk of developing MS by a striking 32-fold, with nearly all individuals diagnosed with MS exhibiting a prior history of EBV infection. These compelling findings establish EBV as a pivotal contributor to the pathogenesis of MS, paving the way for novel preventative and therapeutic strategies targeting the virus (Yamout B. eNeurologicalSci, 2025).

Diagnosing MS requires careful clinical and radiological evaluation. Patients commonly present with symptoms indicative of an inflammatory demyelinating event within the CNS, such as motor weakness, sensory disturbances, or visual impairment. Diagnostic criteria mandate evidence of both dissemination in space (DIS) and dissemination in time (DIT). DIS is confirmed by identifying one or more T2-hyperintense lesions on magnetic resonance imaging (MRI) in at least two of the following CNS regions: periventricular, cortical or juxtacortical, infratentorial, and spinal cord. DIT is evidenced by either the simultaneous presence of enhancing and non-enhancing lesions, the emergence of new T2-hyperintense lesions on follow-up MRI scans, or the detection of oligoclonal bands (OCB) in cerebrospinal fluid, indicative of sustained immune activation. Such meticulous diagnostic approaches enable the early and accurate identification of MS, which is critical for the timely initiation of treatment (Yamout B. eNeurologicalSci, 2025).

MS exhibits a heterogeneous disease course. Approximately 85% of patients initially present with relapsing-remitting MS (RRMS), characterized by episodic relapses followed by periods of partial or complete recovery. Over time, most RRMS patients transition to secondary progressive MS (SPMS), marked by a steady accumulation of disability independent of relapses. In contrast, about 15% of patients experience primary progressive MS (PPMS), characterized by a gradual worsening of neurological function from disease onset, without distinct relapses or remissions (Orbach R, Zhao Z, Wang YC, O'Neill G, Cadavid D. Comparison of disease activity in SPMS and PPMS in the context of multicenter clinical trials. PLoS One. 2012;7(10):e45409).

Emerging evidence underscores the significance of progression independent of relapse activity (PIRA) as a key driver of long-term disability in MS. Notably, PIRA manifests during the early stages of RRMS and reflects the underlying neurodegenerative processes contributing to disease progression. Early, uncontrolled inflammatory activity resulting in axonal damage may diminish brain reserve and impair neuroplasticity, thereby expediting the transition to sustained disability. These findings emphasize the critical importance of prompt and effective suppression of inflammatory disease activity, as evidenced by clinical relapses or the appearance of new or enhancing lesions on MRI (Kappos L, Wolinsky JS, Giovannoni G, et al. Contribution of Relapse-Independent Progression vs Relapse-Associated Worsening to Overall Confirmed Disability Accumulation in Typical Relapsing Multiple Sclerosis in a Pooled Analysis of 2 Randomized Clinical Trials. JAMA Neurol. 2020; 77(9):1132-1140; Pukoli D, Vécsei L. Smouldering Lesion in MS: Microglia, Lymphocytes and Pathobiochemical Mechanisms. Int J Mol Sci. 2023; 24(16)).

Therapeutic management of MS has evolved significantly, with current treatment guidelines prioritizing early intervention, particularly for RRMS. Disease-modifying therapies (DMTs) constitute the cornerstone of MS management. High-efficacy DMTs have demonstrated substantial efficacy in reducing relapse rates and inflammatory activity. However, their impact on halting neurodegeneration or reversing established disability remains limited, particularly in progressive forms of MS (PMS). Nevertheless, initiating treatment early, especially with high-efficacy DMTs, has been associated with improved long-term outcomes, including delaying the transition to SPMS and reducing the likelihood of irreversible disability.

Prognostic factors indicative of an increased risk of disability progression include older age at disease onset, male sex, frequent and severe relapses, incomplete recovery from early relapses, high lesion burden on MRI, and the presence of infratentorial or spinal cord lesions. The identification of these poor prognostic indicators at disease onset serves to guide therapeutic decisions, advocating for the early use of high-efficacy therapies to optimize outcomes.

Currently, there are more than 20 approved therapies for MS, categorized based on efficacy profiles. Moderate-efficacy treatments include interferons, glatiramer acetate, teriflunomide, and dimethyl fumarate. Intermediate-efficacy options encompass fingolimod, siponimod, ponesimod, and ozanimod. High-efficacy therapies, such as natalizumab, ocrelizumab, ofatumumab, ublituximab, alemtuzumab, and cladribine, are reserved for cases warranting aggressive therapeutic intervention. Selecting appropriate treatment based on the presence or absence of poor prognostic factors remains critical in preventing relapses and minimizing disability progression. Through a growing body of evidence, MS is increasingly recognized as a condition requiring personalized therapeutic strategies to address both its relapsing and progressive components (Yamout B. eNeurologicalSci, 2025).

Myelin is an essential lipid-rich, multilamellar membrane that envelops axons, providing electrical insulation and facilitating the unique ion channel configuration required for saltatory conduction of action potentials. This process dramatically increases nerve conduction velocity, enabling rapid and efficient signal transmission along axonal pathways. In the central nervous system (CNS), myelin formation predominantly occurs postnatally and is mediated by oligodendrocytes (OLs), which are specialized, multiprocessed, neuroepithelium-derived glial cells. The population of oligodendrocytes peaks during early developmental stages and remains highly stable across the human lifespan. Despite this stability, new oligodendrocytes continue to emerge throughout adulthood, supporting adaptive myelination in response to changing physiological and environmental demands.

One of the defining biochemical characteristics of myelin is its unusually high lipid-to-protein ratio; over 70% of myelin content consists of lipids, distinguishing it from other cellular membranes. Its lipid composition is uniquely enriched in cholesterol, galactocerebrosides (GaIC), sulfatides, and plasmalogens. Among these, cholesterol is the most abundant lipid, present in both the CNS and peripheral nervous system (PNS). The abundance and distribution of cholesterol within myelin are crucial for its structural integrity and function. Cholesterol synthesis and trafficking in myelinating glial cells are pivotal to the development and maintenance of myelin. Dysregulation of these processes can impair myelin formation and homeostasis.

Cholesterol within the brain can exist in a free form or as cholesterol esters (CEs), which are esterified by the enzyme acyl-coenzyme A cholesterol acyltransferase (ACAT) in the endoplasmic reticulum (ER). The storage of CEs in lipid droplets regulates the balance between free cholesterol (FC) and its esterified form, ensuring dynamic lipid homeostasis within myelinating cells. This finely tuned cholesterol metabolism is especially critical for myelin repair mechanisms.

Lipid mediators play an integral role in modulating autoimmune processes and inflammatory responses, which are central to the pathogenesis of MS. Myelin repair in MS needs an efficient cholesterol metabolism. During demyelinating events, cholesterol is released from damaged myelin, subsequently inhibiting local sterol synthesis through negative feedback mechanisms. This release triggers pro-inflammatory activation of microglia and macrophages, which adopt a phagocytic profile to clear myelin debris. Chronic active MS lesions are characterized by the presence of foamy macrophages containing internalized myelin remnants, indicative of impaired lipid recycling processes.

Although spontaneous remyelination occurs in animal models and likely in the early stages of MS, successful repair requires a conducive regenerative microenvironment. Sterol biosynthesis in microglia and macrophages is essential for creating such an environment, facilitating myelin repair. However, ongoing neuroinflammation and inhibitory lesion-specific factors often impede oligodendrocyte differentiation, ultimately leading to remyelination failure. Detoxifying cholesterol through its conversion into esters and subsequent storage in lipid droplets is a critical prerequisite for effective myelin repair. This process ensures the availability of cholesterol for reuse in remyelination and restoration of myelin integrity.

Despite advancements in understanding the immunopathogenesis of MS, substantial gaps remain regarding the cellular and molecular mechanisms underlying smouldering lesion formation and progression independent of relapse activity (PIRA). Smouldering lesions represent areas of chronic, low-grade inflammation associated with axonal degeneration and sustained neurological decline. Investigating these pathobiochemical markers is crucial for bridging the gap between clinical classification and disease mechanisms in MS. Such insights could inform therapeutic strategies, enabling targeted interventions to address both inflammatory and neurodegenerative components of the disease.

The search for novel biomarkers represents a highly active area of MS research. Biomarkers hold significant promise for elucidating the etiology of MS, refining diagnostic and prognostic frameworks, and advancing the implementation of personalized therapeutic approaches. Lipidomics, the comprehensive study of cellular lipid profiles, has emerged as a vital resource for understanding multifactorial diseases like MS. Pathological conditions induce metabolic reprogramming that alters cellular lipidomes, with such changes often detectable in cerebrospinal fluid (CSF). Given its close interaction with the CNS, CSF reflects the brain's metabolic environment and provides a valuable medium for investigating disease-specific biochemical changes.

In the state of the art, there are few trials that studied the relationship between lipid metabolism ad and MS:
Shah and collaborators examined the levels of cholesterol ester metabolizing enzymes in the brains of patients diagnosed with MS compared to individuals without neurological diseases. The researchers conducted a detailed analysis of brain tissue samples, measuring the concentrations of free and esterified cholesterol alongside the enzymatic activities of cholesterol esterifying and hydrolyzing enzymes. Their findings revealed a notable elevation in cholesterol ester concentrations in the brains of MS patients, which was correlated with a significant reduction in the activity of cholesterol ester hydrolases. A key distinction between the cited study and the disclosure lies in the methodology employed for sample analysis. While the study utilized brain tissue samples to investigate cholesterol ester metabolism, the disclosure focuses on alternative biological matrices, such as CSF or blood, to assess similar biochemical markers. This difference highlights the innovation's potential to provide less invasive diagnostic or prognostic tools for MS, broadening its applicability in clinical settings (Shah SN, Johnson RC. Activity levels of cholesterol ester metabolizing enzymes in brain in multiple sclerosis: correlation with cholesterol ester concentrations. Exp Neurol. 1980 Jun;68(3):601-4).

A research study by Shi and collaborators investigated the role of CSF sphingolipids in distinguishing neuromyelitis optica spectrum disorder (NMOSD) from MS, as well as their correlation with neuroinflammatory cytokines. The research provides an analysis of the concentrations of 25 sphingolipids and 4 lysophosphatidylcholines in patients with varying degrees of NMOSD, MS, and non-inflammatory neurological diseases. One of the key findings disclosed in the study is the inverse correlation between cholesteryl ester 16:0 (CE 16:0) levels and the Expanded Disability Status Scale (EDSS) scores. This correlation suggests that lower levels of CE 16:0 are associated with higher disability scores, indicating its potential as a biomarker for disease severity and progression (Shi L, et al. CSF sphingolipids are correlated with neuroinflammatory cytokines and differentiate neuromyelitis optica spectrum disorder from multiple sclerosis. J Neurol Neurosurg Psychiatry. 2024 Dec 16;96(1):54-67).

Patent publication US 2011152112A1 discloses methods for the prognosis, diagnosis, and prediction of treatment response in diseases associated with deficiencies in the reverse cholesterol transport (RCT) pathway. The disclosed methods involve contacting a population of cells with a modulator specifically designed to influence the RCT pathway and subsequently assessing the lipid efflux profile. This approach provides insights into the functional status of the RCT pathway and its role in disease progression. The parameters investigated in these methods include cholesteryl ester concentration, mRNA expression levels, protein presence and activation states, and phenotypic characteristics of macrophages or macrophage-like cells. By evaluating these parameters, the disclosure enables a comprehensive analysis of lipid metabolism and cellular responses to modulation of the RCT pathway.

The patent document CA2834455A1 focuses on methods related to the prognosis, diagnosis, and treatment of MS. The document emphasizes the role of lipid metabolism and biomarkers in assessing disease progression and therapeutic response. However, the document does not specifically address the use of cholesterol ester levels in CSF, nor does it mention 18:2 cholesteryl ester (18:2 CE) as a biomarker for MS prognosis.

The patent document **CA2782415A1** discloses diagnostic methods for MS that rely on the analysis of biomarkers within biological samples from a patient. The patent describes the identification and application of cholesterol esters, such as cholesterol ester 18:2, as key diagnostic and prognostic biomarkers for MS. The disclosed methods enable the detection of said biomarker levels in patient samples to facilitate the diagnosis of MS and monitor its progression; While the document mentions 18:2 cholesterol ester as one of the biomarkers, it does not include details on CSF or methodologies for detecting any of the biomarker molecules in CSF samples.

MS presents a range of significant unmet needs that hinder effective disease management and patient care. These include the timely detection of early pathological changes, the ability to monitor and address disability progression that occurs independently of relapses, and the accurate assessment of treatment response. A particularly critical challenge in both relapsing MS and progressive MS (PMS) is the prevention of disease worsening despite the use of high-efficacy DMTs.

Currently approved DMTs, while effective at targeting peripheral inflammation and significantly reducing relapse frequency, often fail to adequately address the underlying mechanisms of central nervous system (CNS) compartmentalized inflammation and neurodegeneration. This gap in therapeutic efficacy contributes to ongoing MS progression, which has far-reaching consequences, including substantial personal, social, and professional impacts such as reduced productivity, absenteeism, financial strain, and unemployment. Early intervention aimed at reducing or delaying disease worsening has the potential to significantly improve patients' physical, mental, and cognitive health outcomes, underscoring the need for innovative approaches to MS management.

Research has highlighted a limited window of opportunity in which interventions may effectively slow disease progression, particularly as much of the brain and retinal atrophy associated with MS occurs within the first five years following disease onset. Despite this knowledge, progress in developing effective therapies has been impeded by the absence of validated biomarkers for detecting early worsening and worsening independent of relapses. Additionally, the current lack of sufficiently sensitive measures for evaluating remyelination and axonal regeneration has posed challenges for identifying DMTs capable of mitigating early MS pathology, preventing further deterioration, or promoting recovery.

Thus, there remains an urgent need to develop and validate biomarkers that can accurately identify MS worsening independent of relapses, as well as demonstrate the therapeutic impact of DMTs on disease progression. Addressing these unmet needs could fundamentally enhance the precision and effectiveness of MS treatment, particularly through early intervention strategies.

The disclosure described herein aims to fill this critical gap by leveraging the inventors' findings on lipid profiling as a promising diagnostic and prognostic tool in MS. Specifically, their research highlights the potential utility of 18:2 CE as biomarker for differentiating between various MS disease courses. Quantitative analyses of 18:2 CE levels reveal significant differences when comparing aggressive MS phenotypes to more benign disease trajectories. This innovative lipid profiling approach uncovers previously unrecognized pathological mechanisms within MS, offering valuable insights into disease biology.

Moreover, the identification of 18:2 CE as a biomarker provides an avenue for stratifying patients based on disease severity. Such stratification supports the development of personalized approaches to MS management, encompassing tailored diagnostic methods, refined prognostic assessments, and more targeted therapeutic interventions. By addressing the unmet needs in MS care, this invention has the potential to transform patient outcomes and advance the field of neurology.

With the aim of overcoming the existing drawbacks in the technical field, the present disclosure is provided.

### DESCRIPTION OF THE INVENTION

### General description

The present disclosure provides methods for predicting the prognosis of MS, methods for clasifying an individual based on prognosis, methods for electing a treatment based on prognosis, and kits for performing such claimed methods.

In the PROCOLEM study, which provides the experimental evidence forming the basis of the present disclosure, the inventors evaluated the utility of 18:2 cholesterol ester (18:2 CE) levels in CSF samples obtained from individuals diagnosed with MS at the time of diagnosis. The research aimed to assess the biomarker's potential as a prognostic indicator. This study incorporated a comprehensive 10-year follow-up of CSF samples collected at the point of diagnosis, yielding valuable insights into the long-term applicability and relevance of 18:2 CE as a predictive biomarker.

Historically, neurofilament light chain (NfL) levels in CSF and serum have been widely studied as biomarker for MS and other neurological disorders. NfL reflects acute axonal damage, making it a well-recognized indicator of neurodegenerative activity. However, despite its utility, NfL lacks cell specificity and is elevated in various neurodegenerative conditions, thereby limiting its specificity for MS. Additionally, NfL is not a therapeutic target and provides limited insight into the underlying disease mechanisms. While NfL has demonstrated prognostic value for predicting NEDA-3 status decline within several years of baseline measurements, its predictive capacity diminishes beyond four years post-diagnosis, particularly when high-efficacy DMTs are employed. Another emerging biomarker of interest is glial fibrillary acidic protein (GFAP), which serves as an indicator of astrocyte damage and reactive astrogliosis associated with inflammatory and neurodegenerative diseases. Recent studies have shown that serum GFAP (sGFAP) levels correlate with an increased risk of six-month confirmed disability worsening (CDW) in MS and are linked to 10-year Expanded Disability Status Scale (EDSS) scores. However, efforts to integrate early GFAP testing into predictive models have yielded modest results compared to established clinical and radiological approaches.

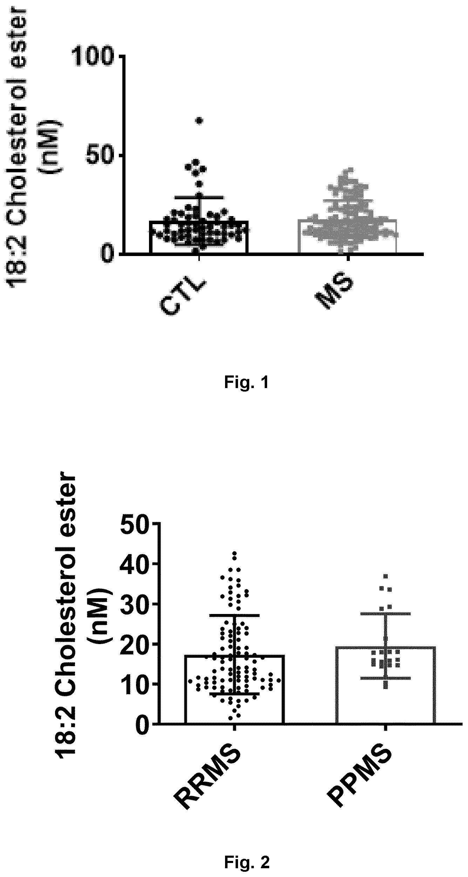

Although the findings indicate that 18:2 CE levels in CSF do not show statistically significant differences between MS patients and controls at the point of diagnosis (Example 4, Figures 1-3), the inventors have demonstrated that 18:2 CE levels serve as a remarkable long-term prognostic biomarker for MS. Given the lack of significant diagnostic differences, a person skilled in the art would likely be discouraged from considering 18:2 CE levels as a prognostic biomarker for MS, particularly over any term. The breakthrough demonstrated by the inventors is the unexpected and exceptional prognostic relevance of 18:2 CE levels over an extended timeframe.

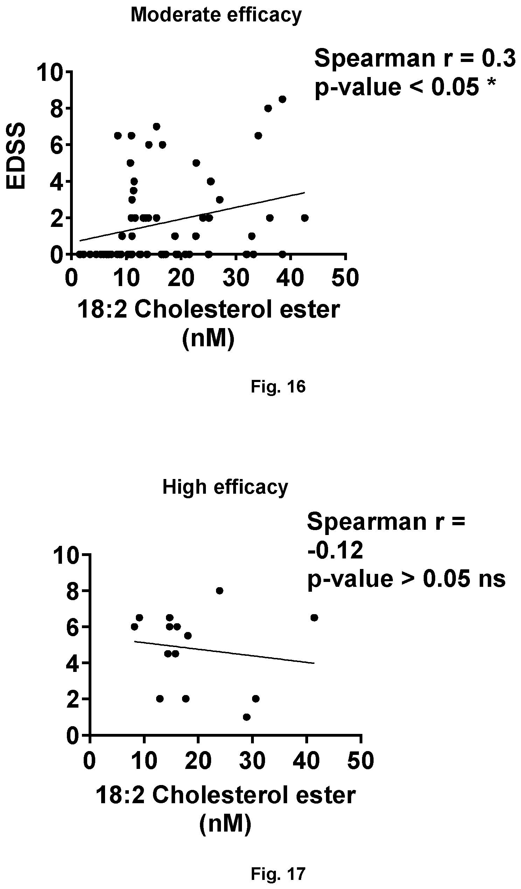

The inventors have also examined the interplay between 18:2 CE levels and the administration of highly effective disease-modifying treatments (DMTs) (Example 7, Figures 16-17). For patients receiving early intervention with high-efficacy DMTs, no correlation was observed between 18:2 CE levels and Expanded Disability Status Scale (EDSS) scores. This finding suggests that early use of such treatments may alter disease prognosis. However, in patients receiving high-efficacy DMTs later in their treatment regimen (as a second or third option), a clear correlation emerged between 18:2 CE levels and EDSS scores. This indicates that elevated 18:2 CE levels could be a valuable prognostic indicator for identifying patients who would benefit from early initiation of high-efficacy treatments to mitigate the risk of long-term disease progression.

The present disclosure is firstly directed in claim 1 to an *in vitro* method for predicting the prognosis of MS in an individual comprising:
(a) quantifying the level of at least one biomarker that is 18:2 cholesterol ester (CE) in a fluid sample from the individual;
(b) means for comparing the quantified level of the biomarker with a reference value; and
(c) assigning a probability of a long-term prognosis of MS in said individual;
wherein the individual has a higher probability of an aggressive course of MS and a higher probability of having evidence of disease activity-3 (EDA-3) when the level of 18:2 CE is above the reference value; and wherein the individual has a higher probability of a mild course of MS and a higher probability of having no evidence of disease activity-3 (NEDA-3) when the level of 18:2 CE is below the reference value.

In a preferred embodiment of the in vitro predicting method disclosed:
- step (a) further comprises registering at least one clinical variable from said individual selected from the group consisting of: Relapse-Associated Worsening (RAW), lesion location, Expanded Disability Status Scale score (EDSS) at diagnosis, Expanded Disability Status Scale score (EDSS) at 3-6 months after diagnosis, oligoclonal band presence (OCB), and any combination thereof;
- step (b) comprises generating a multiple logistic regression model by means of the combination of the quantified 18:2 CE level data with the information from registering at least one clinical variable; and
- step (c) comprises assigning a probability of a 10-year prognosis of MS in said individual;
wherein the multiple regression model is utilized to assign a higher risk of an aggressive course of MS and EDA-3, or higher risk of a mild course of MS and NEDA-3.

According to a particular, preferred embodiment, the sample is a fluid sample selected from the group consisting of: cerebrospinal fluid, blood, serum, and plasma, the cerebrospinal fluid being the most preferred.

Preferably, the step (a) is carried out at disease diagnosis.

A second object of protection is constituted by a method for classifying an individual with MS (see claim 5), comprising:
(a) quantifying the level of at least one biomarker that is 18:2 CE in a sample from said individual; and
(b) means for comparing the quantified level of the biomarker with a reference value; and
(c) classifying the individual for long-term care;
wherein the individual is classified with a mild form of MS when the level of 18:2 CE is below the reference value, and wherein the individual is classified with a severe form of MS when the level of 18:2 CE is above the reference value.

In a preferred embodiment, the in vitro classifying method further comprises the following features:
- step (a) further comprises registering at least one clinical variable from said individual selected from the group consisting of: Relapse-Associated Worsening (RAW), lesion location, Expanded Disability Status Scale score (EDSS) at diagnosis, Expanded Disability Status Scale score (EDSS) at 3-6 months after diagnosis, oligoclonal band presence (OCB), and any combination thereof;
- step (b) comprises generating a multiple logistic regression model by means of the combination of the quantified 18:2 CE level data with the information from registering at least one clinical variable; and
- step (c) comprises classifying the individual for a 10-year term care;
wherein the individual is classified with a mild form of MS or a severe form of MS depending on the multiple regression model data.

A third object of protection is disclosed in claim 7 constituted by an in vitro method for electing a treatment for an individual with MS, comprising:
(a) classifying the individual following the previous classifying method disclosed (claims 5 and 6); and
(b) electing a Disease-Modifying Therapy (DMT) based on the form of MS the individual is suffering from.

According to a preferred embodiment, the elected DMT is a high-efficacy DMT to be administered as first-line when the individual is classified with a severe form of MS and is a naïve patient.

In the most preferred embodiment, the elected DMT is a moderate-efficacy DMT to be administered as first-line when the individual is classified with a mild form of MS and is a naïve patient.

A fourth object of protection is constituted by an *in vitro* kit for predicting the prognosis of MS in an individual according to the previous method, or classifying an individual with MS according to the previopus method, wherein the kit comprises:
- means for quantifying the level of at least one biomarker that is 18:2 CE in a sample from said individual;
- means for registering clinical variables from said individual;
- means for generating a multiple logistic regression model based on the collected data;

- means for assigning a probability of a long-term prognosis of MS in said individual:
- means for classifying said individual with a mild form of MS or a severe form of MS.

A fifth object of protection is the use of this *in vitro* kit for predicting the prognosis of MS in an individual, or classifying an individual with MS, or electing a treatment for an individual with MS, according to the above.

A fifth object of protection is the computer-implemented method for predicting the prognosis of MS in an individual, comprising:
(a) analyzing the data collected through the *in vitro* method for predicting the prognosis of multiple sclerosis (MS) in an individual disclosed above (i.e., at least one biomarker that is 18:2 CE, and at least one clinical variable from said individual), by comparing 18:2 CE level to a reference value, and generating a multiple logistic regression model in combination with the information from registering at least one clinical variable; and
(b) assigning a probability of a long-term prognosis of MS in said individual by means of the multiple regression model, wherein a higher risk of an aggressive course of MS and EDA-3, or higher risk of a mild course of MS and NEDA-3 is assigned.

### Brief description of drawings

**Figure 1** represents the levels of 18:2 CE in CSF samples from patients with MS compared to control subjects at the time of diagnosis.
**Figure 2** represents the comparison of 18:2 CE levels in CSF from patients with RRMS versus patients with PPMS at the time of diagnosis.
**Figure 3** represents the levels of 18:2 CE in CSF from MS patients at diagnosis, comparing samples from patients negative for oligoclonal bands (OCB-) to those positive for oligoclonal bands (OCB+).
**Figure 4** represents 18:2 CE levels in CSF 10 years post-diagnosis, comparing mild MS patients to severe MS patients
**Figure 5** represents 18:2 CE levels in CSF 10 years post-diagnosis, comparing MS patients with EDSS scores ≤ 4 to those with EDSS scores > 4.
**Figure 6** represents the correlation between 10-year follow-up EDSS scores and the levels of 18:2 CE in CSF samples collected at the time of diagnosis.
**Figure 7** represents 18:2 CE levels in CSF 10 years post-diagnosis between MS patients without progression independent of relapse activity (PIRA-) and those with progression independent of relapse activity (PIRA+).
**Figure 8** represents 18:2 CE levels in CSF 10 years post-diagnosis, contrasting MS patients with residual inflammatory activity (RAW-) against those with residual inflammatory activity (RAW+).
**Figure 9** represents 18:2 CE levels in CSF 10 years post-diagnosis, comparing MS patients negative for no evident disease activity (NEDA-3) to those with evident disease activity (EDA-3).
**Figure 10 a)** represents the multiple logistic regression analysis utilizing 18:2 CE levels in conjunction with diagnostic RAW, location of the diagnostic relapse, EDSS scores at diagnosis and six months post-diagnosis, and the presence of OCBs. **Figure 10 b)** represents a 18 CE ROC curve EDA vs. NEDA.
**Figure 11** represents levels of 18:2 CE at year 10 following diagnosis, comparing patients with spinal cord lesions to those without lesions
**Figure 12** represents 18:2 CE levels at year 10 post-diagnosis in patients with supratentorial region lesions compared to patients without lesions.
**Figure 13** represents 18:2 CE levels at year 10 from diagnosis in patients with brainstem lesions versus those without lesions.
**Figure 14** represents 18:2 CE levels at year 10 from diagnosis in patients with cerebellum lesions in comparison to patients without lesions.
**Figure 15** represents 18:2 CE levels at the time of diagnosis, contrasting patients with spinal cord lesions and those without lesions.
**Figure 16** represents the correlation between the EDSS following clinical follow-up and CSF levels of 18:2 CE in patients receiving treatment with moderate-efficacy DMTs.
**Figure 17** represents the correlation between the EDSS after clinical follow-up and CSF levels of 18:2 CE in patients undergoing treatment with high-efficacy DMTs.

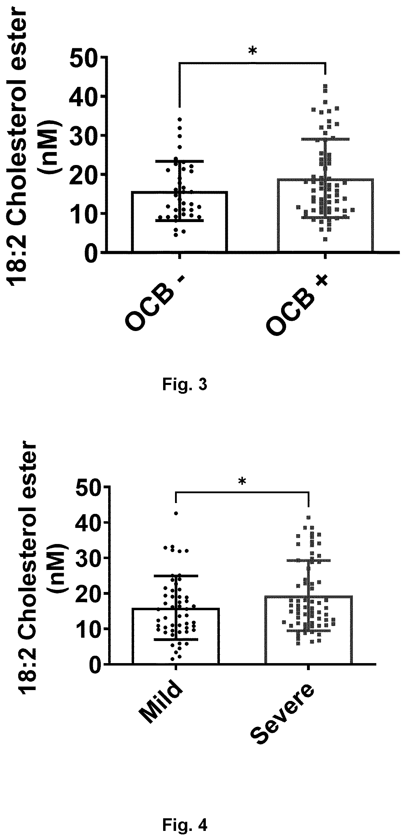

Figures 1-3 present an analysis of 18:2 cholesterol ester (CE) levels in CSF at diagnosis under different conditions. In Figure 1, the data indicate that 18:2 CE levels in the CSF of MS patients do not significantly differ from those observed in controls. Similarly, Figure 2 shows no notable differences in 18:2 CE levels when comparing RRMS patients with PPMS patients at the time of diagnosis. However, Figure 3 demonstrates that within the MS cohort, patients positive for OCB exhibit significantly higher levels of 18:2 CE in CSF compared to OCB-negative patients (*p < 0.05 in unpaired T-TEST). These results suggest a potential link between OCB presence and increased 18:2 CE levels. CE: cholesterol ester; OCB: oligoclonal bands; AU: Arbitrary Units.

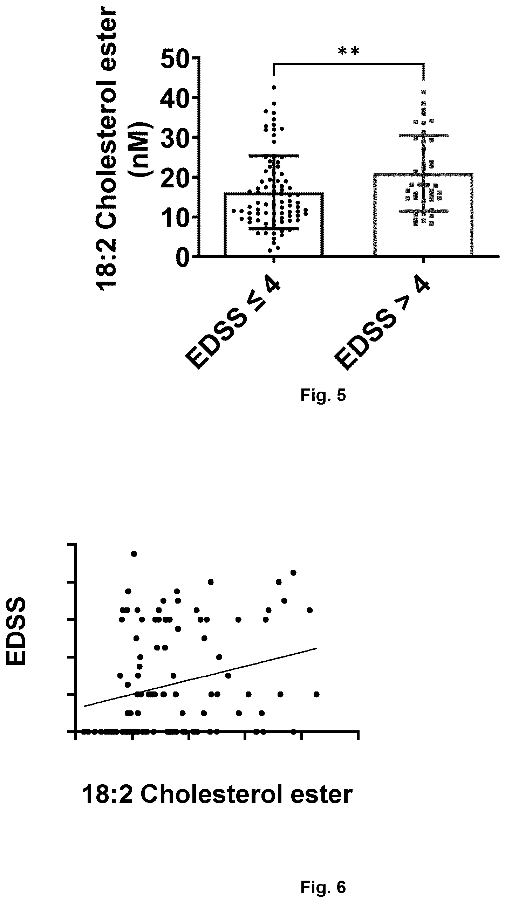

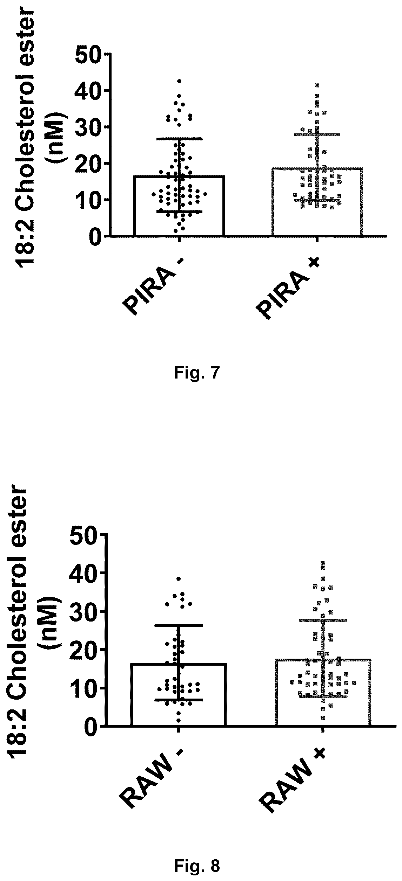

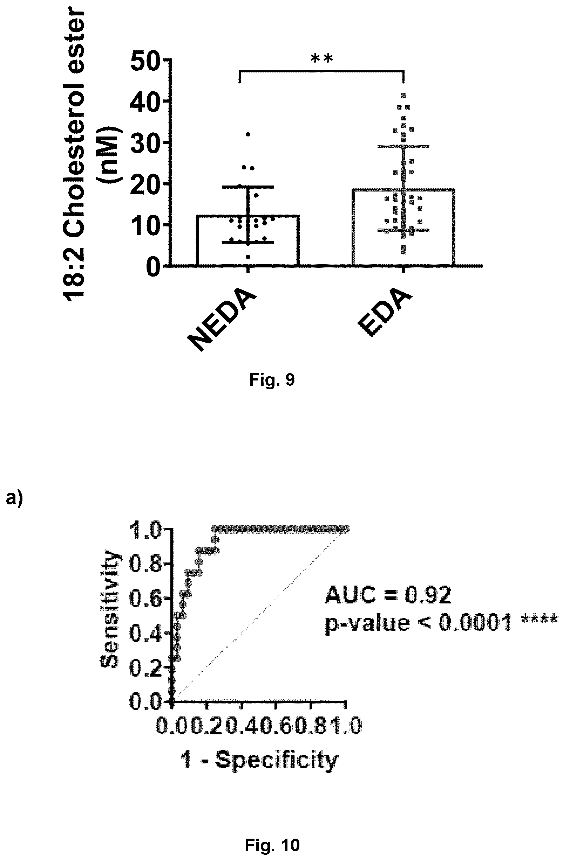

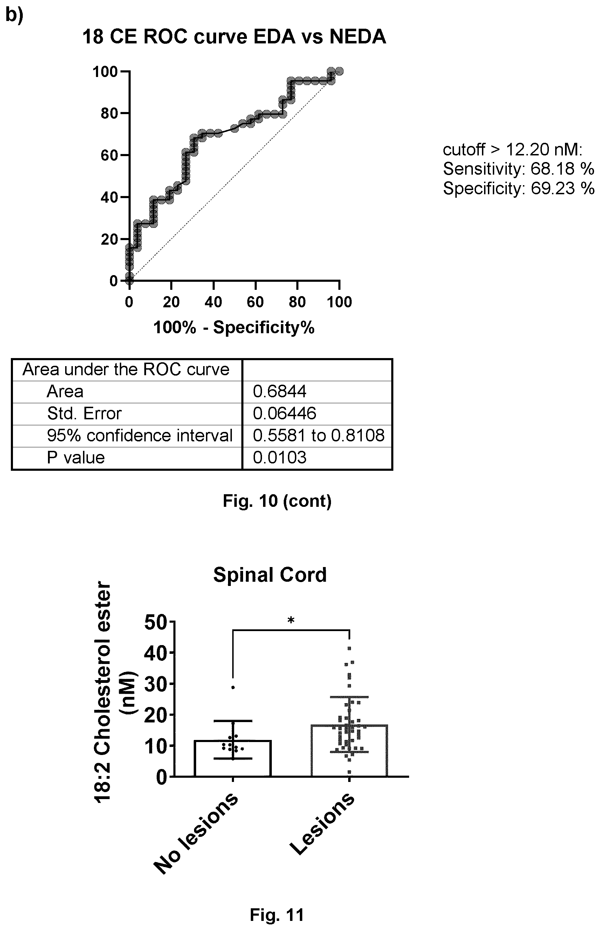

Figures 4-10 explore the relationship between 18:2 CE levels and MS severity over a 10-year follow-up period. Figure 4 shows elevated 18:2 CE levels in CSF observed in patients classified with severe MS compared to those with mild disease progression, suggesting an association between CE levels and disease severity. Figure 5: Patients with EDSS scores > 4 display significantly higher 18:2 CE levels in CSF than those with EDSS ≤ 4, further highlighting the correlation between CE levels and disability progression. Figure 6: A strong correlation is noted between 18:2 CE levels at diagnosis and the 10-year follow-up EDSS scores, supporting its utility as a predictive biomarker for long-term disability. Conversely, in Figure 7: No increase in 18:2 CE levels is detected in patients with PIRA+ compared to PIRA- patients. Similarly, in Figure 8: no elevation in 18:2 CE levels is observed in patients with RAW+ relative to RAW-. Nonetheless, Figure 9 highlights that MS patients with EDA-3 10 years after diagnosis show higher 18:2 CE levels in CSF compared to patients classified as having NEDA-3. Finally, Figure 10 presents the results of a multiple logistic regression analysis, incorporating variables such as 18:2 CE levels, diagnostic residual inflammatory activity (RAW), the location of the diagnostic relapse, EDSS at diagnosis and six months thereafter, and the presence of OCB. This model achieves an area under the curve (AUC) of 0.92 in receiver operating characteristic (ROC) analysis, underscoring the robustness of the combined parameters as predictive indicators. *p < 0.05 in unpaired T-TEST. AU: Arbitrary Units.

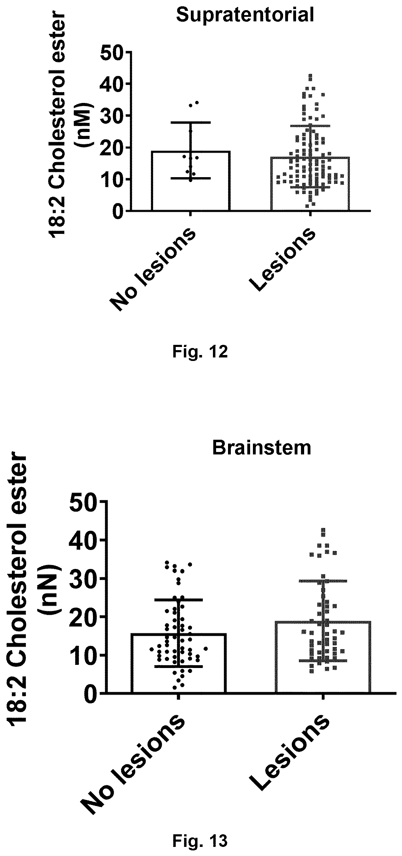

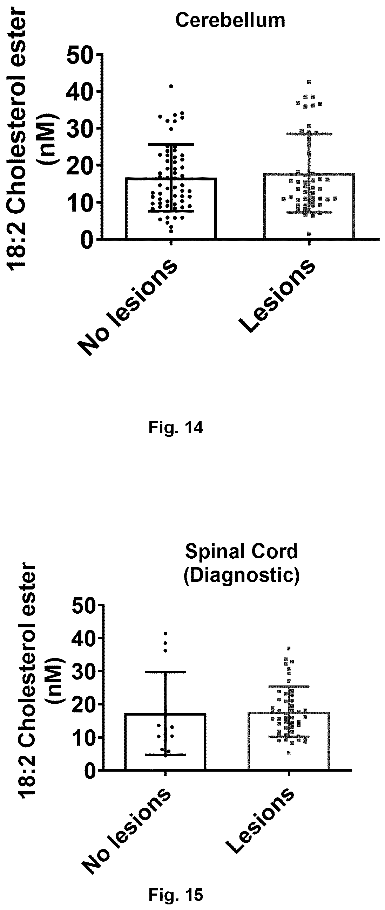

Figures 11-15 investigate the relationship between 18:2 CE levels in CSF and lesion location in MS patients over a 10-year follow-up period. Figure 11 demonstrates a significant association between elevated 18:2 CE levels in CSF and the presence of spinal cord lesions identified during the 10-year follow-up period. This suggests a potential link between 18:2 CE levels and lesion development specifically in the spinal cord. Figure 12 shows that 18:2 CE levels are not significantly associated with lesions in the supratentorial region of the brain. Figure 13 illustrates that no notable association is observed between 18:2 CE levels and lesions in the brainstem. Figure 14 presents similar findings, with no significant correlation between 18:2 CE levels and cerebellar lesions.

Figures 16 and 17 illustrate the correlation between EDSS scores at the 10-year follow-up and 18:2 CE levels in CSF among patients receiving distinct DMTs. Figure 16 demonstrates a statistically significant correlation (*p < 0.05, unpaired T-test) between EDSS scores after the 10-year follow-up and 18:2 CE CSF levels in patients treated with moderate-efficacy DMTs, measured in Arbitrary Units (AU). In contrast, Figure 17 reveals that no such correlation is observed in the patient group receiving high-efficacy DMTs under the same conditions. These results underscore the potential variance in biomarker association depending on treatment efficacy levels.

### EXAMPLES

### Example 1: Study Design and Patients

This retrospective case-control study involved 115 MS patients (21 PPMS and 94 RRMS) diagnosed using 2010 McDonald's criteria, along with 57 control subjects presenting non-demyelinating, non-inflammatory neurological conditions. Cerebrospinal fluid (CSF) samples were obtained at the time of diagnosis, and a minimum follow-up period of 10 years was conducted at Arnau de Vilanova University Hospital.

Patients underwent comprehensive biannual evaluations, including Expanded Disability Status Scale (EDSS) scoring, neuropsychological assessments, and annual magnetic resonance imaging (MRI). Inclusion criteria required adherence to the 2010 McDonald diagnostic criteria, availability of a CSF sample at diagnosis, and EDSS scores validated by a Neurostatus-EDSS-certified physician. Neurologists recorded all relapse events and categorized confirmed and sustained disability worsening (CDW) occurrences into relapse-associated worsening (RAW) or progression independent of relapse activity (PIRA).

Relapses were identified as the onset of new or recurrent MS symptoms persisting for at least 24 hours, documented by the treating neurologist. CDW events were defined by an increase in EDSS scores-≥1.5 points for patients with a baseline EDSS of 0, ≥1.0 points for those with a baseline EDSS of 1-5, and ≥0.5 points for those with a baseline EDSS of ≥5.5-confirmed by a subsequent EDSS assessment conducted three or six months post-worsening onset.

CDW events were classified as follows:
1. Relapse-Associated Worsening (RAW): CDW events occurring within 90 days of an investigator-reported relapse, irrespective of EDSS confirmation.
2. Progression Independent of Relapse Activity (PIRA): CDW events occurring without a prior relapse or with an onset more than 90 days after the last investigator-reported relapse. To qualify as PIRA, no relapse could occur within 30 days before or after EDSS confirmation.

In addition, the study employed the NEDA-3 (No Evidence of Disease Activity-3) criteria, which assess the absence of disease activity across three domains:
- No disability progression (no increase in EDSS).
- No radiological activity (absence of new lesions or enhancement on MRI).

Based on disease severity and progression, MS patients were grouped as follows:
- Mild: EDSS ≤3 after 10 years of disease evolution, no progression in disability except for isolated relapses, and no high-efficacy treatment.
- Severe: EDSS ≥6 after 10 years or requiring high-efficacy treatments over the 10-year period.
- Non-Progressive: Patients with at least 10 years of disease evolution, showing no evidence of progression (EDSS increase of <1 point, excluding relapses; <0.5 points if EDSS ≥6).
- Progressive: Patients with at least 10 years of disease evolution and evidence of progression (EDSS increase >1 point, excluding relapses; >0.5 points if EDSS ≥6).

This study was conducted in compliance with ethical guidelines and approved by the Arnau de Vilanova University Hospital Ethics Committee (Lleida, Spain; CEIC-2719), following the principles of the World Medical Association's Declaration of Helsinki. All participants provided informed consent at the time of lumbar puncture, authorizing the use of their samples for future research purposes.

The clinical characteristics of the participants are described in Table 1:

**Table 1. Description of the participants. RRMS: relapsing-remitting MS; PPMS: primary progressive MS; EDSS: expanded disability severity score; OCB: oligoclonal bands. Data are shown as mean ± SD unless otherwise stated.**

| Patient Characteristic: | All MS, N= 115 | RRMS, N= 94 | PPMS, N= 21 | Controls, N= 57 |
|---|---|---|---|---|
| Age | 39.40 ± 10.23 | 37.81 ± 10.42 | 44.72 ± 8.58 | 40.52 ± 9.31 |
| Sex (% Female) | 77 (66.96) | 70 (74.47) | 7 (33.33) | 41 (71.93) |
| EDSS (diagnostic) | 1.84 ± 1.34 | 1.56 ± 1.26 | 3.19 ± 0.79 | |
| OCB (+/Total) | 63/99 | 50/81 | 13/18 | |

### Example 2: Targeted Lipidomics

18: 2 CE standards were purchased with the C13 isotope (from Merck, Avanti Polar Lipids or similar). A Triple Quad 6420 LC / MS Agilent Technologies (TQD) mass detection system was used for sample analysis. The processing of the samples was carried out by the Lipidomics Service of IRBLleida. Lipid extraction was performed in a single-phase method described previously [27] mixing 10uL of sample with 100uL of butanol: methanol (1:1) containing internal standards. Samples were vortexed thoroughly, sonicated for 1 h, and centrifuged at 14,000xg for 10 min before being analyzed.

Sample analysis was performed by LC-ESI-MS/MS using an Agilent 6495 triple quadrupole mass spectrometer adapting a method described before [28]. Liquid chromatography was performed with a ZORBAX eclipse plus C18 column (2.1x100 mm 1.8 mm, Agilent) heated at 60 °C. Mass spectrometry analysis was performed in positive and negative ion mode with dynamic scheduled multiple reaction monitoring (MRM).

The solvent A consisted in 50% H2O / 30% acetonitrile / 20% isopropanol (v/v/v) and solvent B consisted in 1% H2O / 9% acetonitrile / 90% isopropanol (v/v/v), both containing 10mM ammonium formate. Gradient started with a flow rate of 0.4 ml/min at 10% B and increasing to 45% B over 2.7 min, then to 53% over 0.1 min, to 65% over 6.2 min, to 89% over 0.1 min, to 92% over 1.9 min and finally to 100% over 0.1 min. The solvent was then held at 100% B for 2.3 min. For column equilibration solvent B was decreased from 100% to 10% over 0.1 min and held for an additional 0.9 min; flow rate was then switched to 0.6 ml/min for 1 min before returning to 0.4 ml/min over 0.1 min; and finally solvent B was held at 10% B for a further 0.9 min at 0.4ml/min.

The following mass spectrometer conditions were used: gas temperature 150 °C, gas flow rate 17 L/min, nebulizer 20 psi, sheath gas temperature 200 °C, capillary voltage 3,500 V and sheath gas flow 10 L/min. Isolation widths fort Q1 and Q3 were set to unit resolution (0.7 amu).

### Example 3: Statistical Analyses

All the statistical tests and graphs were performed using GraphPad Prism 9 (GraphPad Software). T-test was employed for two group mean comparison. Correlation analyses were assessed by Spearman's rank. ROC curve was estimated for potential 18:2 CE use of NEDA-3 predictor. Multivariate logistic regression model was performed for NEDA-3 ROC curve of 18:2 CE and clinical variables. The threshold for significance was set at 0.05.

### Example 4: 18:2 CE CSF levels are elevated in BOC + participants

We, the invenors, wanted to explore the potential use of 18:2 CE as a diagnostic biomarker. We did not find statistically significant differences between the controls and the MS patients (Figure 1), including RRMS and PPMS (Figure 2). 18:2 CE was not different between RRMS and PPMS (Figure 2). However, MS patients with OCB+ had higher levels of the 18:2 cholesterol ester in their cerebrospinal fluid than those with OCB- (Figure 3).

### Example 5: 18:2 CE as a long-term prognostic biomarker

When we compared 18:2 CE levels in the total group of MS patients between **mild** (EDSS ≤3 with 10 years of evolution without any progression of disability except for random outbreaks and without high-efficacy treatments) and **severe** (EDSS ≥6 after 10 years of evolution or with high-efficacy treatments over 10 years.) forms, we found significant differences, with higher levels in the latter group (Figure 4).

Next, we attempted to determine whether 18:2 CE levels were different according to EDSS-only criteria for disease severity (≤4 vs >4). We found that patients with an EDSS score of ≤ 4 had lower levels of 18:2 cholesterol ester than those with EDSS scores of >4 (Figure 5). EDSS score after 10-years of follow-up was significantly associated with 18:2 CE levels in CSF at diagnostic point (Figure 6).

We also classified the patients by PIRA in order to check 18:2 CE potential use for its prediction since it could tell us that this lipid might be associated also with relapse independent progression. We did not find differences between PIRA + and PIRA - patients (Figure 7). RRMS patients were also grouped by the presence of RAW but no significant differences were observed (Figure 8).

We also analysed 18:2 CE levels classifying patients by NEDA-3 criteria. In the same line with other prognostic variables, NEDA-3 patients have lower 18:2 CE levels (Figure 9). Since these differences were the highest compared with the other prognostic criteria, we performed a ROC analyses. In a univariate analysis, 18:2 CE alone had an AUC of 0.69 p< 0.05. We envisage a better prognostic power when combined with other variables, all of them around diagnostic time. Therefore, we generated a multiple logistic regression model with 18:2 CE, RAW, lesion location, EDSS at diagnostic and 3-6 months after it and the presence of OCB. This combination reported an AUC of 0.92 p< 0.0001 (Figure 10).

### Example 6: 18:2 CE is elevated in patients with spinal lesions

When we studied 18:2 cholesterol ester levels with respect to the radiological locations of demyelinating lesions, we found that MS patients who did not experience spinal cord injuries had lower levels of 18:2 cholesterol ester than those with spinal cord lesions throughout the 10-year development of the disease (Figure 11). We did not find any statistically significant differences regarding the other territories studied: supratentorial, brainstem, and cerebellum (Figure 12, 13 and 14, respectively). We similarly found no differences regarding whether the relapse that prompted the study was spinal or had another location (Figure 15).

### Example 7: The efficiency of the DMT may interfere with the prognosis

We analysed potential interactions with DMT and the choice of high- or moderate-efficacy and changing from moderate to high-efficacy or viceversa as a second or third option. We evaluated the correlation of EDSS after follow-up an 18:2 CE. We obtained a positive correlation when a moderate efficacy DMT was administered as the first treatment (Figure 16). When the first DMT was high-efficacy, follow-up EDSS and 18:2 CE did not correlate (Figure 17). However, patients treated with a high-efficacy drug as a second or third option showed a positive correlation between 18:2 CE levels and EDSS at 10 years.

## Claims

1. An *in vitro* method for predicting the prognosis of multiple sclerosis (MS) in an individual comprising:
(a) quantifying the level of at least one biomarker that is 18:2 cholesterol ester (CE) in a fluid sample from the individual;
(b) means for comparing the quantified level of the biomarker with a reference value; and
(c) assigning a probability of a long-term prognosis of MS in said individual;
wherein the individual has a higher probability of an aggressive course of MS and a higher probability of having evidence of disease activity-3 (EDA-3) when the level of 18:2 CE is above the reference value; and wherein the individual has a higher probability of a mild course of MS and a higher probability of having no evidence of disease activity-3 (NEDA-3) when the level of 18:2 CE is below the reference value.

2. The *in vitro* method according to claim 1, wherein:
- step (a) further comprises registering at least one clinical variable from said individual selected from the group consisting of: Relapse-Associated Worsening (RAW), lesion location, Expanded Disability Status Scale score (EDSS) at diagnosis, Expanded Disability Status Scale score (EDSS) at 3-6 months after diagnosis, oligoclonal band presence (OCB), and any combination thereof;
- step (b) comprises generating a multiple logistic regression model by means of the combination of the quantified 18:2 CE level data with the information from registering at least one clinical variable; and
- step (c) comprises assigning a probability of a 10-year prognosis of MS in said individual;
wherein the multiple regression model is utilized to assign a higher risk of an aggressive course of MS and EDA-3, or higher risk of a mild course of MS and NEDA-3.

3. The *in vitro* method according to any one of claims 1 or 2, wherein the sample is a fluid sample selected from the group consisting of: cerebrospinal fluid, blood, serum, and plasma.

4. The *in vitro* method according to any one of claims 1 to 3, wherein the step (a) is carried out at disease diagnosis.

5. An *in vitro* method for classifying an individual with MS, comprising:
(a) quantifying the level of at least one biomarker that is 18:2 CE in a sample from said individual; and
(b) means for comparing the quantified level of the biomarker with a reference value; and
(c) classifying the individual for long-term care;
wherein the individual is classified with a mild form of MS when the level of 18:2 CE is below the reference value, and wherein the individual is classified with a severe form of MS when the level of 18:2 CE is above the reference value.

6. The *in vitro* method for classifying an individual with MS according to claim 5, wherein:
- step (a) comprises registering at least one clinical variable from said individual selected from the group consisting of: Relapse-Associated Worsening (RAW), lesion location, Expanded Disability Status Scale score (EDSS) at diagnosis, Expanded Disability Status Scale score (EDSS) at 3-6 months after diagnosis, oligoclonal band presence (OCB), and any combination thereof;
- step (b) comprises generating a multiple logistic regression model by means of the combination of the quantified 18:2 CE level data with the information from registering at least one clinical variable; and
- step (c) comprises classifying the individual for a 10-year term care;
wherein the individual is classified with a mild form of MS or a severe form of MS depending on the multiple regression model data.

7. An *in vitro* method for electing a treatment for an individual with MS, comprising:
(a) classifying the individual according to any one of claims 5 or 6; and
(b) electing a Disease-Modifying Therapy (DMT) based on the form of multiple sclerosis the individual is suffering from.

8. The *in vitro* method according to claim 7, wherein the elected DMT is a high-efficacy DMT to be administered as first-line when the individual is classified with a severe form of multiple sclerosis and is a naïve patient.

9. The *in vitro* method according to claim 7, wherein the elected DMT is a moderate-efficacy DMT to be administered as first-line when the individual is classified with a mild form of MS and is a naïve patient.

10. A kit for the *in vitro* method for predicting the prognosis of MS in an individual according to any one of claims 1-4, or classifying an individual with MS according to any one of claims 5-7, wherein the kit comprises:
- means for quantifying the level of at least one biomarker that is 18:2 CE in a sample from said individual;
- means for registering clinical variables from said individual;
- means for generating a multiple logistic regression model based on the collected data;
- means for assigning a probability of a long-term prognosis of MS in said individual:
- means for classifying said individual with a mild form of MS or a severe form of MS.

11. Use of a kit according to claim 10 for the *in vitro* method for predicting the prognosis of MS in an individual according to any one of claims 1-4, or the *in vitro* method for classifying an individual with MS according to any one of claims 5-6, or the *in vitro* method for electing a treatment for an individual with MS according to any one of claims 7- 9.

12. A computer-implemented method for predicting the prognosis of MS in an individual, comprising:
(a) analyzing the data collected through the *in vitro* method for predicting the prognosis of multiple sclerosis (MS) in an individual according to any one of claims 5-6; by comparing 18:2 CE level to a reference value, and generating a multiple logistic regression model in combination with the information from registering at least one clinical variable; and
(b) assigning a probability of a long-term prognosis of MS in said individual by means of the multiple regression model, wherein a higher risk of an aggressive course of MS and EDA-3, or higher risk of a mild course of MS and NEDA-3 is assigned.
